(19) Europäisches Patentamt

European Patent Office

Office européen des brevets

(11) **EP 1 201 629 B1**

(12) **FASCICULE DE BREVET EUROPEEN**

(45) Date de publication et mention
de la délivrance du brevet:
**15.11.2006 Bulletin 2006/46**

(51) Int Cl.:
*C10G 35/04* [(2006.01)]   *C07C 5/32* [(2006.01)]

(21) Numéro de dépôt: **01402568.8**

(22) Date de dépôt: **03.10.2001**

(54) **Procédé de conversion endothermique d'hydrocarbures, ses utilisations et installation pour la mise en oeuvre de ce procédé**

Verfahren zur endothermischen Umsetzung von Kohlenwasserstoffen, ihre Anwendung und Vorrichtung zur Anwendung dieses Verfahren

Process for the endothermic conversion of hydrocarbons, their uses and installation for application of this process

(84) Etats contractants désignés:
**DE FR GB IT NL**

(30) Priorité: **31.10.2000 FR 0014019**

(43) Date de publication de la demande:
**02.05.2002 Bulletin 2002/18**

(73) Titulaire: **Institut Français du Pétrole**
**92852 Rueil-Malmaison Cedex (FR)**

(72) Inventeurs:
• **Lenglet, Eric**
**92500 Rueil-Malmaison (FR)**
• **Hoffmann, Frédéric**
**69110 Sainte Foy les Lyon (FR)**
• **Boudet, Nicolas**
**69007 Lyon (FR)**

(56) Documents cités:
**US-A- 2 271 646**

## Description

L'industrie pétrolière et pétrochimique utilise de nombreux procédés de conversion d'hydrocarbures par des réactions chimiques endothermiques. On peut notamment citer les réactions de craquage, de déshydrogénation et de réformage d'hydrocarbures.

Ces réactions nécessitent des moyens de chauffage de la charge à des températures élevées pour apporter l'énergie nécessaire à la réaction. Dans de nombreux cas, le chauffage de la charge est suffisant pour atteindre le niveau de température voulu, ainsi que l'énergie nécessaire à la réaction.

Dans d'autres cas, le refroidissement généré par les réactions endothermiques est tel qu'il n'est pas possible de réaliser la réaction chimique dans un seul réacteur. C'est notamment le cas pour le réformage catalytique des hydrocarbures, ou pour la production d'essence à haut indice d'octane.

La charge, typiquement un naphta, additionnée d'hydrogène, traverse successivement plusieurs lits catalytiques, avec des réchauffages entre chacun des lits afin de compenser la baisse de température dans lesdits lits catalytiques, du fait de l'endothermicité de la réaction.

Le plus souvent on utilise plusieurs réacteurs contenant chacun au moins un lit de catalyseur solide ou de façon équivalente des zones catalytiques à catalyseur déposé sur au moins une partie des surfaces solides desdites zones.

Les réchauffages de la charge sont effectués dans des fours tubulaires à radiation, où la charge circule dans des serpentins chauffés dans la zone de radiation alimentée par des brûleurs. On connaît également un procédé de déshydrogénation de paraffines légères, par exemple de propane, pour la production de propylène, où la charge traverse successivement plusieurs réacteurs contenant chacun au moins un lit de catalyseur, avec des réchauffages intermédiaires entre chacun des réacteurs dans des fours tubulaires à radiation.

De façon typique, ces procédés de conversion produisent des composés insaturés qui sont des promoteurs de coke et participent notamment au cokage catalytique. Ce cokage catalytique se produit en particulier dans les fours tubulaires ; en effet la charge qui circule typiquement dans ces fours à des températures d'environ 500 ou 600 °C est soumise au rayonnement des brûleurs dont les gaz de combustion ont typiquement une température comprise entre 1100 et 1600 °C et transmettent des flux thermiques élevés, par exemple de l'ordre de 50 KW/m$^2$ ou plus.

Ces réactions indésirables de cokage dans les fours de réchauffage sont associées également à des réactions de craquage thermique, indésirables. Il en résulte une baisse des rendements de la réaction chimique et des arrêts de l'installation pour le décokage des fours.

Le brevet US-2.271.646 décrit un procédé de craquage catalytique dans des réacteurs, avec des fours de réchauffage intermédiaire (entre deux réacteurs successifs). Il évoque la possibilité d'utiliser également des échangeurs, sans préciser le fluide thermique. Ce brevet mentionne, par ailleurs, la possibilité de récupérer de la chaleur sur des gaz de combustion.

Le brevet US-3.498.755 décrit un procédé de déshydrogénation de l'éthylbenzène dans des zones réactionnelles avec réchauffage intermédiaire au moyen d'un fluide thermique constitué de vapeur d'eau surchauffée. Ce brevet ne décrit pas l'utilisation secondaire de la vapeur d'eau surchauffée dans une application complémentaire.

On connaît également des réacteurs chimiques pour des réactions endothermiques, en particulier celui décrit dans le brevet US-A-5.600.053 où l'apport de chaleur, moins brutal que dans des fours, est réalisé au sein même du réacteur au niveau du lit catalytique. Ces réacteurs, ou réacteurs-échangeurs, chauffent le lit catalytique lui-même, grâce à des surfaces d'échange immergées dans le lit catalytique chauffées par un fluide caloporteur. Dans ce second type de réacteur chimique, le chauffage est moins brutal que dans un four, mais génère cependant des "points chauds" au niveau du catalyseur qui se trouve en contact avec les surfaces chauffantes. Ceci conduit dans de nombreux cas à une désactivation locale du catalyseur, dont la plage de températures d'utilisation est généralement relativement étroite.

Ce second type de réacteur et de procédé de conversion présente donc des inconvénients au niveau de l'utilisation du catalyseur.

L'objet de l'invention est un procédé et une installation de conversion d'hydrocarbures par des réactions endothermiques, supprimant ou limitant de façon importante les phénomènes de cokage catalytique, y compris dans le cas de réactifs promoteur de coke, et ne présentant pas de problèmes d'utilisation du catalyseur, en particulier ne provoquant pas de points chauds au niveau du catalyseur.

Un autre objet de l'invention est un procédé de conversion d'hydrocarbures par des réactions endothermiques, comportant une intégration et une efficacité énergétique élevée.

Un objet particulièrement important de l'invention est un procédé de déshydrogénation d'hydrocarbures à taux de conversion élevée, pour la production d'oléfines. Une utilisation importante de la charge oléfinique formée concerne l'alkylation de composés aromatiques et en particulier la production d'alkylbenzènes (très souvent utilisés comme matière première pour la fabrication de bases de détergents).

Le procédé de la présente invention peut être défini comme un procédé de conversion d'hydrocarbures par au moins une réaction chimique globalement endothermique, dans lequel une charge hydrocarbonée traverse successivement au moins deux zones réactionnelles contenant chacune au moins un catalyseur solide et comprenant entre lesdites zones réactionnelles un réchauffage intermédiaire, dans une zone non catalytique, du courant (ST) issu de la première des deux zones réactionnelles avant son introduction dans ladite seconde zone réactionnelle et dans lequel ce réchauffage est effectué dans un échangeur de chaleur, avec transfert de chaleur essentiellement par convection au moyen d'un fluide thermique TF d'indice de sensibilité au cokage CS inférieur à celui du courant ST, l'écart de température ΔT entre la température du fluide TF à l'entrée de l'échangeur et la température du courant ST à la sortie de l'échangeur de chaleur étant inférieur à environ 250 °C, de préférence inférieur à 200°C.

Ainsi, selon invention le courant de charge partiellement convertie issu par exemple du premier réacteur à lit catalytique est réchauffé essentiellement par convection et non principalement par radiation, c'est-à-dire avec des moyens thermiques beaucoup moins sévères que dans un four de réchauffage à radiation ; l'écart de température ΔT entre le fluide thermique TF chaud à l'entrée de l'échangeur, et le courant ST à la sortie de l'échangeur de chaleur est inférieur à environ 250 °C, de préférence inférieur à 200 °C, et souvent compris entre 10 et 150 °C bornes incluses, et de préférence entre 10 et 120 °C bornes incluses.

Cet écart de température entre les fluides est beaucoup plus modéré que dans un four où les gaz de combustion sont typiquement à des températures supérieures à 1100 °C, donc habituellement supérieures de plus de 500 °C à la température du courant ST du procédé mis en oeuvre et chauffé dans le four.

Ainsi, selon l'invention, on limite considérablement les risques de cokage et la dégradation des réactifs. Par ailleurs ce réchauffage "doux" des réactifs est réalisé sur le fluide seul en sortie d'une zone de réaction, en l'absence de catalyseur. On évite ainsi tous les risques de points chauds au niveau du catalyseur, rencontrés dans les réacteurs-échangeurs catalytiques.

Dans la présente invention, on choisit habituellement un fluide thermique TF relativement peu promoteur de coke, qui peut donc aisément être chauffé dans un four. On transfère ainsi le chauffage relativement brutal sur le fluide thermique et non plus sur la charge réactionnelle.

L'indice de sensibilité au cokage CS est défini conventionnellement pour les fluides TF et ST par la relation :

$$CS = i + 3 \times j$$

dans laquelle :

i est le pourcentage massique de composés monooléfiniques dans le fluide et
j est le pourcentage massique de composés polyinsaturés dans le fluide lesdits composés polyinsaturés comprenant au moins deux liaisons oléfiniques.

A titre d'exemple, pour un fluide contenant 10 % en masse de composés mooléfiniques, et pas de composés polyinsaturés ayant au moins deux liaisons oléfiniques, CS est égal à 10.

Dans le procédé de la présente invention, le fluide thermique TF est la vapeur d'eau sous pression et surchauffée dans des conditions telles qu'il n'y ait pas de condensation, même partielle, de ladite vapeur d'eau. Cette vapeur habituellement typiquement à une température supérieure à environ 400 °C à l'entrée comme à la sortie de l'échangeur de chaleur est utilisée comme un fluide gazeux vecteur de chaleur sensible et non de chaleur latente. Sa pression sera habituellement d'environ 0,1 à environ 15 MPa.

Les fluides thermiques contenant des quantités notables d'oléfines, de dioléfines, et dans une moindre mesure d'aromatiques sont à éviter, car ces composés chimiques sont en effet connus par l'homme du métier comme des promoteurs de coke.

On choisira habituellement un fluide thermique TF d'indice CS inférieur ou égal à 100, souvent d'indice CS inférieur ou égal à 50 et de préférence d'indice CS inférieur ou égal à 10.

Selon un mode de réalisation fréquent la différence de température ΔT est comprise entre 10 °C et 150 °C bornes-incluses, de préférence entre 10 °C et 120 °C bornes incluses.

Souvent le fluide thermique TF est un courant de vapeur d'eau sous une pression supérieure ou égale à environ 0,7 MPa absolus, une partie au moins dudit courant TF en sortie de l'échangeur de chaleur étant détendu dans une turbine pour obtenir une récupération de puissance, en particulier de puissance électrique. Selon une forme de réalisation particulière la dite turbine entraîne un compresseur de recyclage de gaz riche en hydrogène, ce gaz étant ajouté aux hydrocarbures pour effectuer la réaction chimique sous une pression d'hydrogène.

Le procédé de la présente invention peut être utilisé pour la déshydrogénation d'hydrocarbures choisis dans le groupe formé par l'éthane, le propane, le normal butane, l'isobutane, les paraffines comprenant de 5 à 20 atomes de carbone dans leur molécule, les composés monooléfiniques comprenant de 4 à 20 atomes de carbone dans leur molécule et l'éthylbenzène.

Ce procédé est en particulier utilisé pour la déshydrogénation d'une charge de paraffines longues ayant bornes incluses essentiellement entre 10 et 14 atomes de carbone dans leur molécule, pour produire une charge d'alkylation du benzène, dans lequel la charge de paraffines traverse au moins deux zones réactionnelles en présence d'un catalyseur de déshydrogénation, avec réchauffage intermédiaire dans un échangeur de chaleur, permettant d'obtenir une conversion des paraffines d'environ 15 à 50 % en poids. Dans le cadre de cette utilisation pour la production d'une charge d'alkylation du benzène le nombre de zones réactionnelles est de préférence compris entre 2 et 6, les températures de ces zones sont généralement comprises entre 440 et 520 °C et le rapport molaire d'hydrogène par rapport aux hydrocarbures dans ces zones est généralement compris entre 1 : 1 et 15:1.

Une autre utilisation fréquente du procédé selon l'invention est le réformage sous hydrogène des hydrocarbures.

Selon une forme particulière de réalisation du procédé de l'invention le courant de fluide thermique TF est composé de vapeur d'eau sous une pression d'environ 1,5 MPa à environ 13 MPa absolus, et une partie au moins de ce courant TF, en sortie de l'échangeur, est utilisée, en condensation, pour le rebouillage d'une colonne de fractionnement par distillation.

Dans l'utilisation du procédé de l'invention dans une réaction d'alkylation par le benzène des oléfines produites par la déshydrogénation des paraffines, en présence de benzène, en excès, on prévoit une étape de fractionnement dans une colonne de distillation de l'effluent de l'étape d'alkylation pour la séparation du benzène en excès des composés moins volatils, comprenant des paraffines non transformées et des alkylbenzènes, dans laquelle une partie au moins du courant TF, en sortie dudit échangeur est utilisée pour le rebouillage de la dite colonne de distillation.

La présente invention concerne aussi une installation pour la réalisation du procédé décrit ci-devant dans lequel l'échangeur utilisé est un échangeur à plaques, en particulier un échangeur à plaques en acier inoxydables.

Selon une forme particulière on utilise pour la mise en oeuvre du procédé de la présente invention un fluide thermique TF qui est un courant de vapeur d'eau à une pression P1, dont une partie est recomprimée en aval de l'échangeur de chaleur, par un éjecteur à vapeur alimenté par un courant de vapeur à une pression P2 supérieure à P1, pour être recyclé, au moins en partie en amont du dit échangeur.

La figure 1 représente une partie d'une installation de conversion d'hydrocarbures pour la réalisation du procédé selon l'invention.
La figure 2 représente une partie d'une installation de conversion d'hydrocarbures pour la réalisation d'une première variante du procédé selon l'invention.
La figure 3 représente une partie d'une installation de conversion d'hydrocarbures pour la réalisation d'une seconde variante de réalisation du procédé selon l'invention.
La figure 4 représente une partie d'un complexe chimique pour la production d'alkylbenzènes, comprenant une étape de déshydrogénation de paraffines longues, conforme au procédé de l'invention.

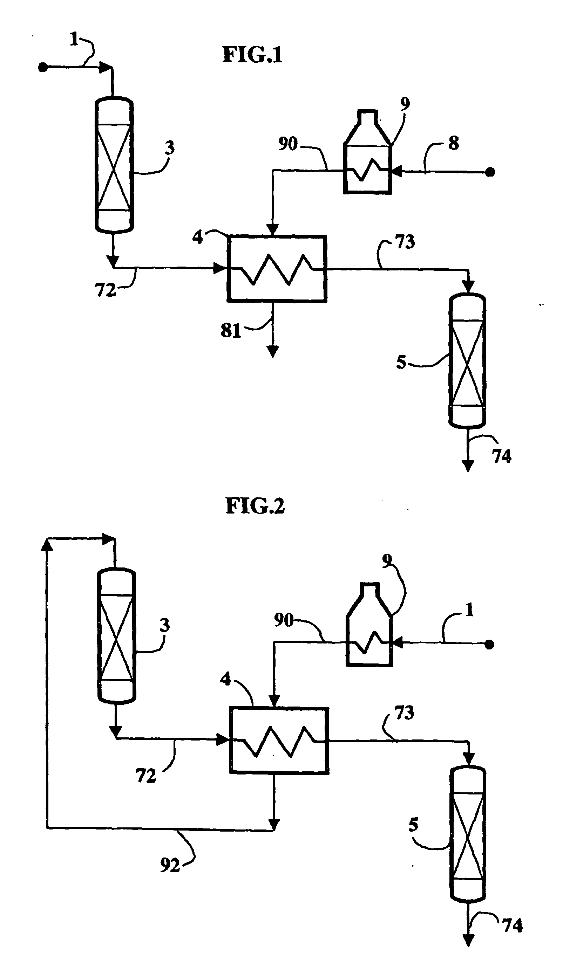

On se réfère maintenant à la figure 1. Un courant préchauffé circulant dans la ligne (1) et contenant une charge d'hydrocarbures traverse un premier lit de catalyseur dans le réacteur (3), ce qui permet d'obtenir un courant ST contenant la charge partiellement convertie envoyée par la ligne (72) dans l'échangeur de chaleur (4). La réaction étant endothermique, le courant ST circulant dans la ligne (72) en sortie du réacteur (3) a une température inférieure à celle du courant entrant dans le réacteur (3) par la ligne (1). Ce courant ST est chauffé par échange thermique indirect, essentiellement par convection, dans l'échangeur de chaleur (4), afin de remonter sa température à un niveau suffisant. Le courant ST réchauffé est envoyé par la ligne (73) dans le réacteur (5) contenant un lit de catalyseur pour obtenir une conversion supplémentaire de la charge issue du réacteur (3). On récupère le produit issu du réacteur (5) par la ligne (74). Un courant comprenant essentiellement des composés chimiques relativement peu cokants ou non cokants, est envoyé par le conduit (8) puis chauffé dans un four tubulaire (9). Il alimente ensuite l'échangeur de chaleur (4), par le conduit (90) en tant que fluide thermique TF et ressort de cet échangeur par le conduit (81). La différence de température $\Delta T$ entre la température de ce fluide TF dans le conduit (90) à son entrée dans l'échangeur (4) et la température du fluide ST circulant dans le conduit (73) à la sortie de l'échangeur (4) est choisie relativement faible, par exemple de 10 à 100 °C, de façon à sensiblement éliminer les risques de cokage dans l'échangeur (4).

On se réfère maintenant à la figure 2, utilisant les mêmes références que la figure 1.

Dans cette variante de réalisation de l'invention, c'est un courant circulant dans la ligne (1) et contenant la charge, ou par exemple la charge d'hydrocarbures additionnée de gaz de recyclage riche en hydrogène qui constitue le fluide thermique chaud TF, après chauffage dans le four (9). Le courant TF est envoyé par la ligne (90) dans l'échangeur (4). En sortie de l'échangeur (4) il alimente alors par la ligne (92) le premier réacteur (3). Le reste de l'installation est identique à celui décrit en liaison avec la description de la figure 1.

Sur ces deux figures 1 et 2, on a schématisé uniquement deux étages catalytiques ; dans la pratique, on pourra utiliser un nombre plus élevé de réacteurs, ou zones catalytiques, par exemple de 3 à 10 zones catalytiques, avec des réchauffages intermédiaires. Une partie au moins de ces réchauffages est réalisée conformément à l'invention.

Les lits catalytiques sont du type à catalyseur solide. La taille des particules de catalyseur, la géométrie des lits catalytiques (radiaux, axiaux ou autres) ne constituent pas des limitations de l'invention qui peut être mise en oeuvre avec tous types de lits catalytiques : lit fixe, lit mobile, lit fluide, catalyseur déposé en surface de solides (couches minces), par exemple réacteurs monolithes.

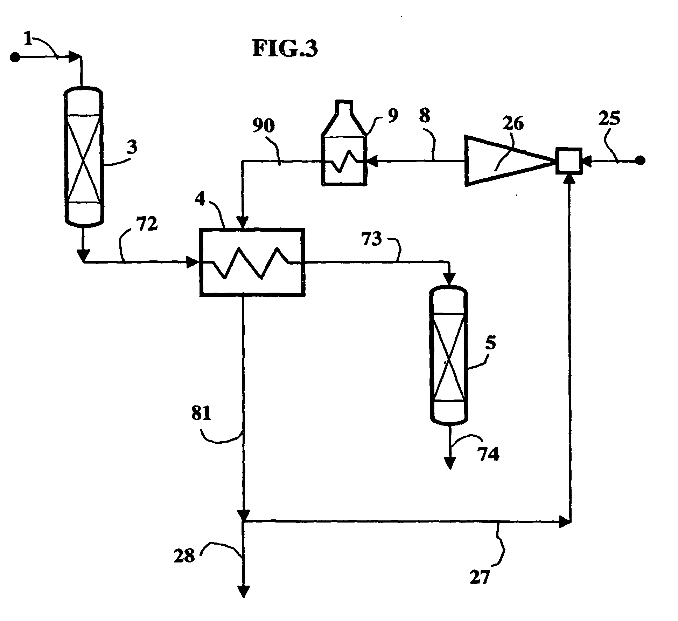

On se réfère maintenant à la figure 3, utilisant les mêmes références que la figure 1.

Une partie de l'installation de la figure 3 est identique à celle de la figure 1 ; dans la figure 3, le fluide thermique TF est constitué de vapeur d'eau, par exemple de la vapeur à moyenne pression P1 (1 à 2,5 MPa par exemple) ; en sortie de l'échangeur (4) une partie du fluide thermique TF est purgée par la ligne (28) (utilisée pour d'autres besoins thermiques, ou de génération de puissance mécanique ou électrique, ces moyens n'étant pas représentés) ; une autre partie du courant TF est envoyée par la ligne (27) puis recomprimée dans l'éjecteur à vapeur (26) alimenté par de la vapeur haute pression entrant par la ligne (25) dans cet éjecteur, par exemple à une pression P2 comprise entre 10 et 13 MPa absolus.

En sortie de l'éjecteur (26), le courant de vapeur est envoyé par la ligne (8) et chauffé dans le four (9) pour constituer le fluide thermique chaud TF envoyé par la ligne (90) dans l'échangeur de chaleur (4).

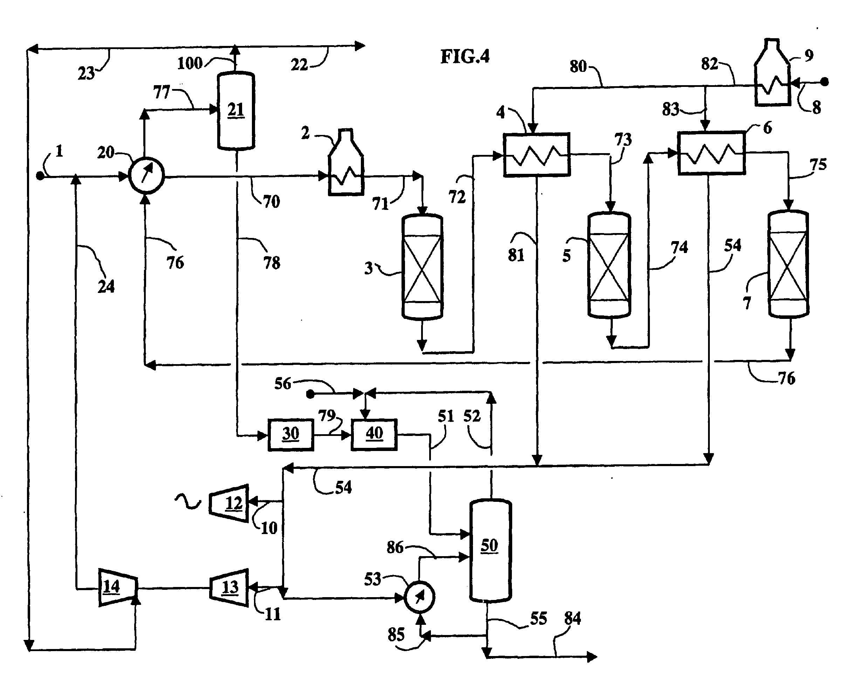

On se réfère maintenant à la figure 4. Cette figure représente une partie d'un complexe pétrochimique pour la production d'alkylbenzènes, notamment linéaires, connus de l'homme de l'art sous le sigle "LAB" (de l'anglais Linear Alkyl Benzène).

Le courant circulant dans la ligne (1) riche en hydrocarbures de la charge, essentiellement des paraffines notamment linéaires ayant de 10 à 14 atomes de carbone, et additionné d'un courant, arrivant par la ligne (24), de gaz de recyclage riche en hydrogène, est préchauffé dans l'échangeur (20), surchauffé à la température réactionnelle, par exemple 485 °C dans le four (2) dans lequel il arrive par ligne (70), avant d'alimenter par ligne (71) le premier réacteur de déshydrogénation catalytique (3). En sortie du réacteur (3), le courant ST contenant la charge partiellement convertie est envoyé par la ligne (72) puis réchauffé selon l'invention dans l'échangeur de chaleur (4). Il alimente ensuite par la ligne (73) le second réacteur de déshydrogénation catalytique (5). En sortie du réacteur (5), le fluide réactionnel formé est envoyé par la ligne (74) puis réchauffé dans l'échangeur de chaleur (6), (lui aussi selon l'invention), avant d'être envoyé par la ligne (75) dans le troisième réacteur de déshydrogénation à lit catalytique (7).

En sortie du réacteur (7) l'effluent de la section de déshydrogénation catalytique est envoyé par la ligne (76) dans l'échangeur (20) puis en sortie de cet échangeur il est envoyé par la ligne (77) dans un ballon séparateur gaz/liquide (21). Le gaz produit dans le ballon (21) sort par la ligne (100) dudit ballon puis est séparé en un excès de gaz riche en hydrogène évacué par la ligne (22), et en un courant de gaz de recyclage envoyé par la ligne (23) puis recomprimé par le compresseur (14) pour constituer le courant envoyé par la ligne (24) dans la ligne (1) d'arrivée de la charge pour former le mélange charge-gaz de recyclage mentionné ci-devant.

Le circuit du fluide thermique est constitué à partir d'un courant de vapeur d'eau arrivant par la ligne (8) (par exemple à 40 bars absolus au niveau des échangeurs de chaleur selon l'invention). Ce courant de vapeur d'eau est chauffé dans le four tubulaire à radiation (9), pour constituer un fluide thermique TF permettant d'alimenter les échangeurs de chaleur (4) et (6) respectivement par les lignes (80) et (83).

En aval des échangeurs de chaleur (4) et (6), le fluide thermique TF (vapeur moyenne pression), partiellement refroidi, alimente trois réseaux distincts :

- le fluide envoyé dans le premier réseau, respectivement par les lignes (81) et (54), alimenté par la ligne (10) est détendu dans une turbine (12) pour la production d'énergie électrique.

- Le fluide envoyé dans le second réseau par la ligne (11) est détendu dans la turbine (13) qui entraîne le compresseur de gaz de recyclage (14).

- Le fluide envoyé dans le troisième réseau par la ligne (54), alimente et sert de fluide thermique au rebouilleur à condensation (53) d'une colonne de distillation (50). Cette colonne (50) est alimentée par le courant d'effluent liquide de la section de déshydrogénation, à partir du ballon séparateur (21) par la ligne (78) ; ce courant liquide issu du ballon (21) traverse successivement une unité d'hydrogénation sélective (des dioléfines) (30), puis via la ligne (79) une unité d'alkylation (40) par le benzène. Cette unité est alimentée par du benzène via la ligne (56) additionné par du benzène recyclé via la ligne (52). Ainsi la colonne (50) est la première colonne de fractionnement de l'effluent de l'unité d'alkylation (40), et est alimentée par la ligne (51). Cette colonne (50) réalise le fractionnement du benzène en excès (courant recyclé par la ligne (52)) des produits moins volatils contenant notamment des paraffines non transformées et des alkylbenzènes évacués par la ligne (84). Cette colonne (50) comporte un rebouilleur (53) alimenté par la ligne (85) dont l'effluent réchauffé est renvoyé dans la colonne (50) par la ligne (86).

Ce schéma est très performant au niveau énergétique car la fluide thermique TF, utilisé pour les réchauffages intermédiaires de la section de déshydrogénation, est également utilisé de façon intégrée dans le complexe en aval de ces échangeurs pour la production d'énergie électrique et/ou d'énergie mécanique et/ou et d'énergie thermique.

Les lits catalytiques sont par exemple (sans que cela limite l'invention) des lits catalytiques radiaux, contenant un catalyseur de déshydrogénation par exemple de type platine/étain sur un support alumine sous forme de billes. Les températures à l'entrée des lits catalytiques peuvent typiquement être de l'ordre de 420 à 550 °C , préférentiellement de 450 à 500 °C.

Le taux molaire d'hydrogène par rapport aux hydrocarbures est typiquement compris entre 1 et 15, de préférence entre 2 et 10, et la pression à la sortie du dernier réacteur est typiquement comprise entre 0,10 MPa (méga pascals) et 5 MPa absolus bornes incluses, et de préférence entre 0,15 MPa et 0,5 MPa absolus bornes incluses.

Les vitesses spatiales (VVH, vitesse volumique horaire) peuvent varier typiquement pour chaque lit catalytique entre 10 h$^{-1}$ et 250 h$^{-1}$.

L'installation peut éventuellement comprendre non pas 3 zones catalytiques, comme indiqué sur le schéma de la figure 4, mais par exemple 4 ou 5 zones catalytiques, avec des réchauffages intermédiaires, voire plus si nécessaire.

Une telle installation peut permettre d'atteindre, en fonction des températures utilisées notamment, des conversions très élevées, comprises par exemple entre 15 % et 50 %, en particulier entre 18 % et 35 % en masse de la charge de paraffines employée, sans risques notables de cokage, et sans points chauds au niveau du catalyseur.

## Revendications

1. Procédé de conversion d'hydrocarbures par au moins une réaction chimique globalement endothermique du groupe constitué par a) le réformage sous hydrogène, et b) la déshydrogénation d'hydrocarbures choisis dans le groupe formé par l'éthane, le propane, le normal butane, l'isobutane, les paraffines comprenant de 5 à 20 atomes de carbone dans leur molécule, les composés monooléfiniques comprenant de 4 à 20 atomes de carbone dans leur molécule, l'éthylbenzène, et les mélanges d'au moins deux de ces hydrocarbures, dans lequel une charge hydrocarbonée traverse successivement au moins deux zones réactionnelles contenant chacune au moins un catalyseur solide et comprenant entre lesdites zones réactionnelles un réchauffage intermédiaire, dans une zone non catalytique, du courant (ST) issu de la première des deux zones réactionnelles avant son introduction dans la deuxième zone réactionnelle **caractérisé en ce que** ce réchauffage est effectué dans un échangeur de chaleur, avec transfert de chaleur essentiellement par convection au moyen d'un fluide thermique TF constitué de vapeur d'eau surchauffée vecteur de chaleur sensible, de température supérieure à 400°C à rentrée et à la sortie dudit échangeur, **en ce que** ce fluide thermique TF est chauffé dans un four en amont de l'échangeur de chaleur, **en ce que** l'écart de température $\Delta T$ entre la température du fluide TF à l'entrée de l'échangeur et la température du courant ST à la sortie de l'échangeur de chaleur est inférieur à 250 °C,

et **en ce que** le courant TF de vapeur d'eau en sortie de l'échangeur est utilisé selon l'une des utilisations suivantes:

    - A) dans une turbine de détente, TF ayant une pression supérieure ou égale à 0.7 MPa, aux fins de récupération de puissance.
    - B) en condensation, pour le rebouillage d'une colonne de fractionnement par distillation, TF ayant une pression comprise entre environ 1,5 et 13 MPa.
    - C) par recyclage dans ledit échangeur, après recompression d'une partie de TF depuis une pression P1 au moyen d'un éjecteur à vapeur alimenté par un courant de vapeur d'eau à une pression P2> P1.

2. Procédé selon la revendication 1 dans lequel le fluide thermique TF est un courant de vapeur d'eau sous une pression supérieure ou égale à environ 0,7 MPa absolus, une partie au moins dudit courant TF en sortie de l'échangeur de chaleur est détendu dans une turbine pour obtenir une récupération de puissance, ladite turbine entraînant un compresseur de recyclage de gaz riche en hydrogène, ce gaz étant ajouté aux hydrocarbures pour effectuer la réaction chimique sous une pression d'hydrogène.

3. Utilisation du procédé selon l'une des revendications 1 et 2 pour la déshydrogénation d'une charge de paraffines longues ayant bornes incluses essentiellement entre 10 et 14 atomes de carbone dans la molécule, pour produire une charge d'alkylation du benzène, dans lequel la charge de paraffines traverse au moins deux zones réactionnelles en présence d'un catalyseur de déshydrogénation, avec réchauffage intermédiaire dans une échangeur de chaleur, permettant d'obtenir une conversion des paraffines d'environ 15 à 50 % en poids.

4. Utilisation du procédé selon la revendication 3 dans lequel le nombre de zones réactionnelles est compris entre 2 et 6, les températures de ces zones sont comprises entre 440 et 520 °C et le rapport molaire d'hydrogène par rapport aux hydrocarbures dans ces zones est compris entre 1 : 1 et 15 : 1.

5. Utilisation du procédé selon les revendications 3 et 4, comprenant une étape d'alkylation par le benzène des oléfines produites par la déshydrogénation des paraffines, en présence de benzène en excès, et une étape de fractionnement dans une colonne de distillation de l'effluent de l'étape d'alkylation pour la séparation du benzène en excès des composés moins volatils, comprenant des paraffines non transformées et des alkylbenzènes, dans lequel une partie au moins du courant TF, en sortie dudit échangeur est utilisée pour le rebouillage de ladite colonne de distillation.

6. Installation pour la réalisation du procédé selon l'une des revendications précédentes , dans lequel l'échangeur est un échangeur à plaques, en particulier un échangeur à plaques en acier inoxydables.

## Claims

1. A process for converting hydrocarbons using at least one globally endothermic chemical reaction from the group formed by a) the reforming under hydrogen, and b) the dehydrogenation of hydrocarbons from the group formed by ethane, propane, normal butane, isobutane, paraffins comprising from 5 to 20 carbon atoms in their molecule, ethylbenzene, and the mixtures of at least two from the precited hydrocarbons, in which a hydrocarbon feed successively traverses at least two reaction zones each containing at least one solid catalyst and comprising between said reaction zones an intermediate reheating step, in a non catalytic zone, for reheating the stream (ST) from the first of the two reaction zones prior to its introduction into said second reaction zone, and in which said reheating is carried out in a heat exchanger, with heat transfer essentially by convection using a thermal fluid TF which is constituted by superheated steam as a sensible heat medium, with a temperature over 400°C at the inlet and at the outlet of said heat exchanger, wherein said thermal fluid TF is heated in a furnace upstream of the heat exchanger, and in that the difference in temperature $\Delta T$ between the temperature of the fluid TF at the inlet to the exchanger and the temperature of the stream ST at the heat exchanger outlet being less than 250°C, and wherein said steam stream TF at the exchanger outlet is used according to one of the following options:

- a) in an expansion turbine, TF pressure being over or equal to 0.7 MPa, for the purpose of power recovery,
- b) as a condensation fluid for reboiling a distillation fractionation column, TF pressure being between 1.5 and 13 MPa,
- c) through recycling into said heat-exchanger, after compression of a portion of TF from a pressure P1 using a steam ejector fed by a steam stream at a pressure P2> P1.

**2.** A process according to claim 1, in which the thermal fluid TF is a stream of steam at a pressure of 0.7 MPa absolute or more, at least a portion of said stream TF at the outlet from the heat exchanger being depressurised in a turbine to generate power, in which said turbine drives a compressor for recycling a hydrogen-rich gas, this gas being added to hydrocarbons to carry out the chemical reaction under hydrogen pressure.

**3.** Use of a process according one of claims 1 and 2, to dehydrogenate a feed of long chain paraffins essentially containing 10 to 14 carbon atoms per molecule, limits included, to produce a benzene alkylation feed, in which the paraffin feed traverses at least two reaction zones in the presence of a dehydrogenation catalyst, with intermediate reheating in a heat exchanger, to obtain a paraffin conversion of about 15% to 50% by weight.

**4.** Use of a process according to claim 3, in which the number of reaction zones is in the range 2 to 6, the temperatures of these zones are in the range 440°C to 520°C and the mole ratio of hydrogen with respect to the hydrocarbons in these zones is in the range 1:1 to 15:1.

**5.** Use of a process according one of claims 3 and 4, comprising a step using benzene for alkylating olefins produced by paraffin dehydrogenation, in the presence of excess benzene, and a step for fractionating the effluent from the alkylation step in a distillation column to separate excess benzene from less volatile compounds comprising non transformed paraffins and alkylbenzenes, in which at least a portion of stream TF from the outlet from said exchanger is used to reboil said distillation column.

**6.** A unit for carrying out the process of any one of claims 1 to 13, in which the exchanger used is a plate heat exchanger, in particular a plate heat exchanger with stainless steel plates.


**Patentansprüche**

**1.** Verfahren zur Umwandlung von Kohlenwasserstoffen, bei dem Kohlenwasserstoffe, die aus der Gruppe gewählt werden, die aus Ethan, Propan, n-Butan, Isobutan, Paraffinen, deren Molekül 5 bis 20 Kohlenstoffatome umfasst, mono-olefinischen Verbindungen, deren Molekül 4 bis 20 Kohlenstoffatome umfasst, Ethylbenzol und Mischungen mindestens zweier dieser Kohlenwasserstoffe besteht, mindestens einer insgesamt endothermen chemischen Reaktion aus einer Gruppe, die aus a) dem Reformieren unter Wasserstoff und b) dem Dehydrieren von Kohlenwasserstoffen besteht, unterworfen werden, wobei eine Kohlenwasserstoffcharge nacheinander mindestens zwei Reaktionsbereiche durchläuft, die beide mindestens einen feststofflichen Katalysator enthalten, und sich zwischen den Reaktionsbereichen, in einem nichtkatalytischen Bereich, eine Vorrichtung befindet, den Stoffstrom (ST) aus dem ersten Reaktionsbereich erneut aufzuheizen, bevor dieser in den zweiten Reaktionsbereich gelangt, **dadurch gekennzeichnet, dass** das erneute Aufheizen in einem Wärmetauscher durchgeführt wird, wobei die Wärmeübertragung hauptsächlich mittels eines flüssigen Trägers TF freier Wärme erfolgt, der aus überhitzten Wasserdampf besteht, welcher am Eingang und am Ausgang des Tauschers eine Temperatur von mehr als 400 °C aufweist, und dass der flüssige Wärmeträger TF in einem Ofen erwärmt wird, bevor er in den Wärmetauscher gelangt, und dass der Temperaturunterschied $\Delta T$ zwischen der Temperatur des Trägers TF am Eingang des Tauschers und der Temperatur des Stroms ST am Ausgang des Tauschers weniger als 250 °C beträgt, und dass Wasserdampfstrom TF am Ausgang des Tauschers gemäß einer der folgenden Anwendungen verwendet wird:

- A) in einer Entspannungsturbine, wobei TF einen Druck von mindestens 0,7 MPa ausweist, und zwar zum Zwecke der Leistungsrückgewinnung,
- B) im Kondensationsverfahren, zum Erhitzen einer Fraktionierkolonne mit einem Aufkocher, wobei TF einen Druck zwischen 1,5 und 13 MPa aufweist,
- C) Durch Rückführung in den Tauscher, nachdem ein Teil von TF, der einen Druck P1 aufweist, mittels einer Dampfstrahlpumpe, die mit einem Dampfstrom, der unter einem Druck P2 > P1 steht, betrieben wird, erneut komprimiert worden ist.

2. Verfahren nach Anspruch 1, wobei es sich bei dem flüssigen Wärmeträger TF um Dampfdampf handelt, der unter einem Absolutdruck von mindestens 0,7 MPa steht, und mindestens ein Teil des TF-Stroms am Ausgang des Tauschers in einer Turbine entspannt wird, um eine Leistungsrückgewinnung zu erzielen, und die Turbine einen Kompressor zur Rückführung wasserstoffreichen Gases antreibt, wobei dieses Gas den Kohlenwasserstoffen zugeführt wird, um die chemische Reaktion unter Wasserstoffdruck durchzuführen.

3. Anwendung des Verfahren nach einem der Ansprüche 1 oder 2 zur Dehydrierung einer Paraffincharge, um eine Charge zur Benzolalkylierung herzustellen, wobei die Moleküllänge der Paraffine im Wesentlichen auf 10 bis 14 Kohlenstoffatome beschränkt ist, die Paraffincharge mindestens zwei Reaktionsbereiche in Gegenwart eines Dehydrierungskatalysators durchläuft und dazwischen in einem Wärmetauscher erneut aufgeheizt wird, so dass die Paraffine zu ungefähr 15 bis 50 Gewichtsprozent umgewandelt werden können.

4. Anwendung des Verfahrens nach Anspruch 3, wobei die Anzahl der Reaktionsbereiche zwischen 2 und 6 beträgt, die Temperaturen dieser Bereiche zwischen 440 und 520 °C liegen und das Molverhältnis des Wasserstoffs gegenüber den Kohlenwasserstoffen in diesen Bereiche zwischen 1:1 und 15:1 beträgt.

5. Anwendung des Verfahren nach den Ansprüchen 3 und 4, einen Alkylierungsschritt umfassend, in welchem die Olefine, die bei der Dehydrierung der Paraffine hergestellt wurden, mit Benzol umgesetzt werden, sowie einen Schritt, in welchem der aus dem Alkylierungsschritt stammende Stoffstrom einer Destillation in einer Fraktionierkolonne unterzogen wird, um das überschüssige Benzol von den weniger flüchtigen Verbindungen, welche nicht umgewandelte Paraffine sowie Alkylbenzole umfassen, zu trennen, wobei mindestens ein Teil des TF-Stroms am Ausgang des Tauschers zum Erhitzen dieser Fraktionierkolonne mit einem Aufkocher verwendet wird.

6. Anlage zur Durchführung des Verfahrens nach einem der vorhergehenden Ansprüche, in welcher es sich bei dem Tauscher um einen Plattentauscher handelt, insbesondere um einen Tauscher mit Edelstahlplatten.

**FIG.1**

**FIG.2**

# FIG.3

FIG.4